# EUROPEAN PATENT SPECIFICATION

(11) **EP 1 861 421 B1**
(45) Date of publication and mention of the grant of the patent: **19.10.2011**
(21) Application number: 06725046.4
(22) Date of filing: 14.03.2006
(51) Int. Cl.: C07K 14/435

(54) **Recombinant expression of insect defensins in Aspergillus**
REKOMBINANTE EXPRESSION VON INSEKTEN DEFENSINEN IN ASPERGILLUS
Expression recombinée de défensines d'insectes dans Aspergillus

(30) Priority: 16.03.2005 DK 200500375
(43) Date of publication of application: 05.12.2007
(73) Proprietor: Novozymes Adenium Biotech A/S, 2880 Bagsvaerd (DK)
(72) Inventor: HOEGENHAUG, Hans-Henrik, Kristensen, DK-2840 Holte (DK); SCHNORR, Kirk, Matthew, DK-2840 Holte (DK); HANSEN, Mogens, Trier, DK-3540 Lynge (DK)
(74) Representative: Jensen, Bo Hammer
(86) International application number: PCT/EP2006/060695
(87) International publication number: WO 2006/097464

(56) References cited:
- WO-A-03/044049
- WO-A-2006/050737
- XU DEPING ET AL: "Systemic induction of a potato pin2 promoter by wounding, methyl jasmonate, and abscisic acid in transgenic rice plants" PLANT MOLECULAR BIOLOGY, vol. 22, no. 4, 1993, pages 573-588, XP008062560 ISSN: 0167-4412
- MYGIND P H ET AL: "Plectasin is a peptide antibiotic with therapeutic potential from a saprophytic fungus" NATURE, NATURE PUBLISHING GROUP, LONDON, GB, vol. 437, no. 7061, October 2005 (2005-10), pages 975-980,969, XP002376185 ISSN: 0028-0836

### SEQUENCE LISTING

<110> Novozymes A/S
<120> Recombinant Expression of Defensins in Filamentous Fungi
<130> 10789.204-WO
<160> 24
<170> PatentIn version 3.3
<210> 1
   <211> 288
   <212> DNA
   <213> Pseudoplectania nigrella
<220>
   <221> CDS
   <222> (1)..(285)
<220>
   <221> sig_peptide
   <222> (1) .. (69)
<220>
   <221> mat_peptide
   <222> (166)..(285)
<400> 1
   atg caa ttt acc acc atc ctc tcc atc ggt atc acc gtc ttc gga ctt       48
   Met Gln Phe Thr Thr Ile Leu Ser Ile Gly Ile Thr Val Phe Gly Leu
   -55                 -50                 -45                 -40
   ctc aac acc gga gcc ttt gca gca ccc cag cct gtt ccc gag gct tac       96
   Leu Asn Thr Gly Ala Phe Ala Ala Pro Gln Pro Val Pro Glu Ala Tyr
                   -35                 -30                 -25
   gct gtt tct gat ccc gag gct cat cct gac gat ttt gct ggt atg gat      144
   Ala Val Ser Asp Pro Glu Ala His Pro Asp Asp Phe Ala Gly Met Asp
               -20                 -15                 -10
   gcg aac caa ctt cag aaa cgt gga ttt gga tgc aat ggt cct tgg gat      192
   Ala Asn Gln Leu Gln Lys Arg Gly Phe Gly Cys Asn Gly Pro Trp Asp
           -5              -1  1               5
   gag gat gat atg cag tgc cac aat cac tgc aag tct att aag ggt tac      240
   Glu Asp Asp Met Gln Cys His Asn His Cys Lys Ser Ile Lys Gly Tyr
   10                  15                  20                  25
   aag gga ggt tat tgt gct aag ggg ggc ttt gtt tgc aag tgt tac tag      288
   Lys Gly Gly Tyr Cys Ala Lys Gly Gly Phe Val Cys Lys Cys Tyr
                   30                  35                  40
<210> 2
   <211> 95
   <212> PRT
   <213> Pseudoplectania nigrella
<400> 2
   Met Gln Phe Thr Thr Ile Leu Ser Ile Gly Ile Thr Val Phe Gly Leu
   -55                 -50                 -45                 -40
   Leu Asn Thr Gly Ala Phe Ala Ala Pro Gln Pro Val Pro Glu Ala Tyr
                   -35                 -30                 -25
   Ala Val Ser Asp Pro Glu Ala His Pro Asp Asp Phe Ala Gly Met Asp
               -20                 -15                 -10
   Ala Asn Gln Leu Gln Lys Arg Gly Phe Gly Cys Asn Gly Pro Trp Asp
           -5              -1  1               5
   Glu Asp Asp Met Gln Cys His Asn His Cys Lys Ser Ile Lys Gly Tyr
   10                  15                  20                  25
   Lys Gly Gly Tyr Cys Ala Lys Gly Gly Phe Val Cys Lys Cys Tyr
                   30                  35                  40
<210> 3
   <211> 273
   <212> DNA
   <213> Eurotium amstelodami
<220>
   <221> CDS
   <222> (1)..(270)
<220>
   <221> sig_peptide
   <222> (1) .. (60)
<220>
   <221> mat_peptide
   <222> (145)..(270)
<400> 3
   atg cac ttc acc aag gtc tcc acc att ctt ttt acc atc ttc gcc gcc        48
   Met His Phe Thr Lys Val Ser Thr Ile Leu Phe Thr Ile Phe Ala Ala
               -45                 -40                 -35
   ggc atc atg gct gct ccc acc gaa gga gtc cgt gag gaa gcc gcc cct        96
   Gly Ile Met Ala Ala Pro Thr Glu Gly Val Arg Glu Glu Ala Ala Pro
           -30                 -25                 -20
   ggc cag gag gtt tac ccc gac gaa cct cct gct tct ctg acc aag cgt       144
   Gly Gln Glu Val Tyr Pro Asp Glu Pro Pro Ala Ser Leu Thr Lys Arg
       -15                 -10                 -5                  -1
   ggc ttc gga tgt cct ggt gat gcc tac cag tgc agt gaa cac tgc agg       192
   Gly Phe Gly Cys Pro Gly Asp Ala Tyr Gln Cys Ser Glu His Cys Arg
   1               5                   10                  15
   gcc ctg ggc ggt gga cgc act gga gga tac tgt gct gga cct tgg tat       240
   Ala Leu Gly Gly Gly Arg Thr Gly Gly Tyr Cys Ala Gly Pro Trp Tyr
               20                  25                  30
   ttg ggt cac cct acc tgc acc tgt tct ttc taa                           273
   Leu Gly His Pro Thr Cys Thr Cys Ser Phe
           35                  40
<210> 4
   <211> 90
   <212> PRT
   <213> Eurotium amstelodami
<400> 4
   Met His Phe Thr Lys Val Ser Thr Ile Leu Phe Thr Ile Phe Ala Ala
               -45                 -40                 -35
   Gly Ile Met Ala Ala Pro Thr Glu Gly Val Arg Glu Glu Ala Ala Pro
           -30                 -25                 -20
   Gly Gln Glu Val Tyr Pro Asp Glu Pro Pro Ala Ser Leu Thr Lys Arg
       -15                 -10                 -5              -1
   Gly Phe Gly Cys Pro Gly Asp Ala Tyr Gln Cys Ser Glu His Cys Arg
   1               5                   10                  15
   Ala Leu Gly Gly Gly Arg Thr Gly Gly Tyr Cys Ala Gly Pro Trp Tyr
               20                  25                  30
   Leu Gly His Pro Thr Cys Thr Cys Ser Phe
           35                  40
<210> 5
   <211> 273
   <212> DNA
   <213> Picea glauca
<220>
   <221> CDS
   <222> (1)..(270)
<220>
   <221> sig_peptide
   <222> (1)..(60)
<220>
   <221> mat_peptide
   <222> (151)..(270)
<400> 5
   atg aag ttc acc atc tcc atc atc gcc gct ctc gct ttc ttc gcc cag        48
   Met Lys Phe Thr Ile Ser Ile Ile Ala Ala Leu Ala Phe Phe Ala Gln
   -50                 -45                 -40                 -35
   gga atc gtg gca gct cct gcg cct atc ccc gag gcc gcc gcg gtg gct       96
   Gly Ile Val Ala Ala Pro Ala Pro Ile Pro Glu Ala Ala Ala Val Ala
                   -30                 -25                 -20
   gcc cca gag gcc gag cca aag gcg tta gat gag ctt ccg gag ttg caa      144
   Ala Pro Glu Ala Glu Pro Lys Ala Leu Asp Glu Leu Pro Glu Leu Gln
               -15                 -10                 -5
   aag cgt ggc ttt ggg tgc aac ggt tgg cct ttc gag gac gat gag cag      192
   Lys Arg Gly Phe Gly Cys Asn Gly Trp Pro Phe Glu Asp Asp Glu Gln
       -1  1               5                   10
   tgc cat aat cac tgc aag acc att cct ggt tat aag ggt ggc tac tgt      240
   Cys His Asn His Cys Lys Thr Ile Pro Gly Tyr Lys Gly Gly Tyr Cys
   15                  20                  25                  30
   gcc aac gtt ggc acc act tgc aag tgc tac tag                          273
   Ala Asn Val Gly Thr Thr Cys Lys Cys Tyr
                   35                  40
<210> 6
   <211> 90
   <212> PRT
   <213> Picea glauca
<400> 6
   Met Lys Phe Thr Ile Ser Ile Ile Ala Ala Leu Ala Phe Phe Ala Gln
   -50                 -45                 -40                 -35
   Gly Ile Val Ala Ala Pro Ala Pro Ile Pro Glu Ala Ala Ala Val Ala
                   -30                 -25                 -20
   Ala Pro Glu Ala Glu Pro Lys Ala Leu Asp Glu Leu Pro Glu Leu Gln
               -15                 -10                 -5
   Lys Arg Gly Phe Gly Cys Asn Gly Trp Pro Phe Glu Asp Asp Glu Gln
       -1  1               5                   10
   Cys His Asn His Cys Lys Thr Ile Pro Gly Tyr Lys Gly Gly Tyr Cys
   15                  20                  25                  30
   Ala Asn Val Gly Thr Thr Cys Lys Cys Tyr
                   35                  40
<210> 7
   <211> 189
   <212> DNA
   <213> Crassostrea virginica
<220>
   <221> CDS
   <222> (1)..(186)
<220>
   <221> sig_peptide
   <222> (1) .. (66)
<220>
   <221> mat_peptide
   <222> (67)..(186)
<400> 7
   atg aaa gtg ttt gta ctt ctt aca ata gca gta atg ctt atg gta tct       48
   Met Lys Val Phe Val Leu Leu Thr Ile Ala Val Met Leu Met Val Ser
           -20                 -15                 -10
   gcc gac gtt gct ttg gcc ggc ttt ggc tgt cct ttg aac cgg tac cag       96
   Ala Asp Val Ala Leu Ala Gly Phe Gly Cys Pro Leu Asn Arg Tyr Gln
       -5              -1  1               5                   10
   tgt cac tca cat tgc caa tcc att ggt cgt aaa ggt gga tac tgt ggt      144
   Cys His Ser His Cys Gln Ser Ile Gly Arg Lys Gly Gly Tyr Cys Gly
                   15                  20                  25
   ggg tgg tgg agt ttt aca tgc aca tgc tac cgc act aaa aag tag          189
   Gly Trp Trp Ser Phe Thr Cys Thr Cys Tyr Arg Thr Lys Lys
               30                  35                  40
<210> 8
   <211> 62
   <212> PRT
   <213> Crassostrea virginica
<400> 8
   Met Lys Val Phe Val Leu Leu Thr Ile Ala Val Met Leu Met Val Ser
           -20                 -15                 -10
   Ala Asp Val Ala Leu Ala Gly Phe Gly Cys Pro Leu Asn Arg Tyr Gln
       -5              -1  1               5                   10
   Cys His Ser His Cys Gln Ser Ile Gly Arg Lys Gly Gly Tyr Cys Gly
                   15                  20                  25
   Gly Trp Trp Ser Phe Thr Cys Thr Cys Tyr Arg Thr Lys Lys
               30                  35                  40
<210> 9
   <211> 189
   <212> DNA
   <213> Mesobuthus gibbosus
<220>
   <221> CDS
   <222> (1)..(186)
<220>
   <221> sig_peptide
   <222> (1) .. (72)
<220>
   <221> mat_peptide
   <222> (73)..(186)
<400> 9
   atg aaa acc att gta ctt ctt ttc gtg ttg gct tta gta ttc tgc act        48
   Met Lys Thr Ile Val Leu Leu Phe Val Leu Ala Leu Val Phe Cys Thr
                   -20                 -15                 -10
   ctt gaa atg gga atg gtg gaa gcc gga ttc ggt tgt cca ttc aat caa        96
   Leu Glu Met Gly Met Val Glu Ala Gly Phe Gly Cys Pro Phe Asn Gln
               -5                  -1  1               5
   gga aga tgt cac aga cat tgt cga agt att cgt cga aga gga gga tat       144
   Gly Arg Cys His Arg His Cys Arg Ser Ile Arg Arg Arg Gly Gly Tyr
       10                  15                  20
   tgc gat gga ttt ttg aaa caa aga tgt gtt tgt tat cgg aga taa           189
   Cys Asp Gly Phe Leu Lys Gln Arg Cys Val Cys Tyr Arg Arg
   25                  30                  35
<210> 10
   <211> 62
   <212> PRT
   <213> Mesobuthus gibbosus
<400> 10
   Met Lys Thr Ile Val Leu Leu Phe Val Leu Ala Leu Val Phe Cys Thr
                   -20                 -15                 -10
   Leu Glu Met Gly Met Val Glu Ala Gly Phe Gly Cys Pro Phe Asn Gln
               -5                  -1  1               5
   Gly Arg Cys His Arg His Cys Arg Ser Ile Arg Arg Arg Gly Gly Tyr
       10                  15                  20
   Cys Asp Gly Phe Leu Lys Gln Arg Cys Val Cys Tyr Arg Arg
   25                  30                  35
<210> 11
   <211> 198
   <212> DNA
   <213> Crassostrea gigas
<220>
   <221> CDS
   <222> (1)..(195)
<220>
   <221> sig_peptide
   <222> (1)..(66)
<220>
   <221> mat_peptide
   <222> (67)..(195)
<400> 11
   atg aaa gta ttc gtt ctt tta aca cta gct gtc ctt ctg atg gtt tct      48
   Met Lys Val Phe Val Leu Leu Thr Leu Ala Val Leu Leu Met Val Ser
           -20                 -15                 -10
   gca gac atg gct ttt gct gga ttt ggg tgt ccg ggt aac cag tta aag      96
   Ala Asp Met Ala Phe Ala Gly Phe Gly Cys Pro Gly Asn Gln Leu Lys
       -5              -1  1               5                   10
   tgc aac aat cac tgc aag tcc att agt tgt aga gcg ggc tac tgt gat     144
   Cys Asn Asn His Cys Lys Ser Ile Ser Cys Arg Ala Gly Tyr Cys Asp
                   15                  20                  25
   gca gcc acg ctc tgg tta aga tgt aca tgt acc gat tgt aat gga aag     192
   Ala Ala Thr Leu Trp Leu Arg Cys Thr Cys Thr Asp Cys Asn Gly Lys
               30                  35                  40
   aag taa                                                             198
   Lys
<210> 12
   <211> 65
   <212> PRT
   <213> Crassostrea gigas
<400> 12
   Met Lys Val Phe Val Leu Leu Thr Leu Ala Val Leu Leu Met Val Ser
           -20                 -15                 -10
   Ala Asp Met Ala Phe Ala Gly Phe Gly Cys Pro Gly Asn Gln Leu Lys
       -5              -1  1               5                   10
   Cys Asn Asn His Cys Lys Ser Ile Ser Cys Arg Ala Gly Tyr Cys Asp
                   15                  20                  25
   Ala Ala Thr Leu Trp Leu Arg Cys Thr Cys Thr Asp Cys Asn Gly Lys
               30                  35                  40
   Lys
<210> 13
   <211> 195
   <212> DNA
   <213> Crassostrea virginica
<220>
   <221> CDS
   <222> (1) .. (192)
<220>
   <221> sig_peptide
   <222> (1) .. (66)
<220>
   <221> mat_peptide
   <222> (67)..(192)
<400> 13
   atg aaa gtg ttt gtt ctt cta aca ata gct gtc atg ctt ttg gta tct      48
   Met Lys Val Phe Val Leu Leu Thr Ile Ala Val Met Leu Leu Val Ser
       -20                 -15                 -10
   gcc gat gta gct act gca gat aac gga tgt ccc cgt cgt ccg aga atc      96
   Ala Asp Val Ala Thr Ala Asp Asn Gly Cys Pro Arg Arg Pro Arg Ile
       -5              -1  1               5                   10
   tgt cac aat cgg tgc ata tac aaa ggt cgt aga ggc gga aaa tgt gtc     144
   Cys His Asn Arg Cys Ile Tyr Lys Gly Arg Arg Gly Gly Lys Cys Val
                   15                  20                  25
   gga aag tgg aga agc tta tgc gaa tgc atc tac cca tcg aag gcc ggg     192
   Gly Lys Trp Arg Ser Leu Cys Glu Cys Ile Tyr Pro Ser Lys Ala Gly
               30                  35                  40
   tga                                                                 195
<210> 14
   <211> 64
   <212> PRT
   <213> Crassostrea virginica
<400> 14
   Met Lys Val Phe Val Leu Leu Thr Ile Ala Val Met Leu Leu Val Ser
       -20                 -15                 -10
   Ala Asp Val Ala Thr Ala Asp Asn Gly Cys Pro Arg Arg Pro Arg Ile
       -5              -1  1               5                   10
   Cys His Asn Arg Cys Ile Tyr Lys Gly Arg Arg Gly Gly Lys Cys Val
                   15                  20                  25
   Gly Lys Trp Arg Ser Leu Cys Glu Cys Ile Tyr Pro Ser Lys Ala Gly
               30                  35                  40
<210> 15
   <211> 207
   <212> DNA
   <213> Aspergillus oryzae
<220>
   <221> CDS
   <222> (1) .. (207)
<220>
   <221> sig_peptide
   <222> (1) .. (54)
<220>
   <221> mat_peptide
   <222> (79) .. (207)
<400> 15
   atg aaa ctt ctg acg gtc gcc ttt tcc ctt ctt ctt ctc ggg caa gtc       48
   Met Lys Leu Leu Thr Val Ala Phe Ser Leu Leu Leu Leu Gly Gln Val
       -25                 -20                 -15
   cat gcc agt cct ttg gta ctc gac aaa agg tct tcc tgc cag ttg ggt       96
   His Ala Ser Pro Leu Val Leu Asp Lys Arg Ser Ser Cys Gln Leu Gly
   -10                 -5                  -1  1               5
   gac gtc tgg gac ctc aat gct gca gac gcc gcc tgc agc gct tcg tgt      144
   Asp Val Trp Asp Leu Asn Ala Ala Asp Ala Ala Cys Ser Ala Ser Cys
               10                  15                  20
   gcc att caa cac ggc gac aaa cac ggc gga cac tgc gat aag aac aag      192
   Ala Ile Gln His Gly Asp Lys His Gly Gly His Cys Asp Lys Asn Lys
           25                  30                  35
   gtc tgc gtc tgc aat                                                  207
   Val Cys Val Cys Asn
       40
<210> 16
   <211> 69
   <212> PRT
   <213> Aspergillus oryzae
<400> 16
   Met Lys Leu Leu Thr Val Ala Phe Ser Leu Leu Leu Leu Gly Gln Val
       -25                 -20                 -15
   His Ala Ser Pro Leu Val Leu Asp Lys Arg Ser Ser Cys Gln Leu Gly
   -10                 -5                  -1  1               5
   Asp Val Trp Asp Leu Asn Ala Ala Asp Ala Ala Cys Ser Ala Ser Cys
               10                  15                  20
   Ala Ile Gln His Gly Asp Lys His Gly Gly His Cys Asp Lys Asn Lys
           25                  30                  35
   Val Cys Val Cys Asn
       40
<210> 17
   <211> 362
   <212> DNA
   <213> Artificial
<220>
   <223> Pleactasin encoding sequence with intron (see Example 1)
<400> 17
   ggatccacca tgcaatttac caccatcctc tccatcggta tcaccgtctt cggacttctc      60
   aacaccggag cctttgcagc accccagccg gtacccgagg cttacgctgt ttctgatccc     120
   gaggctcatc ctgacgattt tgctggtatg gatgcgaacc aacttcagaa acgtggattt     180
   ggatgcaatg gtccttggga tgaggatgat atgcagtgcc acaagtaaga atcacttata     240
   actagtagat taagccaaga gtattggaac tgatgataaa tagtcactgc aagtctatta     300
   agggttacaa gggaggttat tgtgctaagg ggggctttgt ttgcaagtgt tactagctcg     360
   ag                                                                    362
<210> 18
   <211> 303
   <212> DNA
   <213> Artificial
<220>
   <223> Intron-less MluN1 containing sequence encoding Plectasin (see
   Example 3)
<400> 18
   ggatccacca tgcaatttac caccatcctc tccatcggta tcaccgtctt cggactggcc     60
   aacaccggag cctttgcagc accccagccg gtacccgagg cttacgctgt ttctgatccc    120
   gaggctcatc ctgacgattt tgctggtatg gatgcgaacc aacttcagaa acgtggattt    180
   ggatgcaatg gtccttggga tgaggatgat atgcagtgcc acaatcactg caagtctatt    240
   aagggttaca agggaggtta ttgtgctaag gggggctttg tttgcaagtg ttactagctc    300
   gag                                                                  303
<210> 19
   <211> 358
   <212> DNA
   <213> Artificial
<220>
   <223> Plectasin encoding sequence with intron of pMT2899 (see Example
   3)
<400> 19
   ggatccacca tgcaatttac caccatcctc tccatcggta tcaccgtctt cggactgggt     60
   atgtacacca cccccttgcg tctgatctgt gacatatgta gctgactggt cagccaacac    120
   cggagccttt gcagcacccc agccggtacc cgaggcttac gctgtttctg atcccgaggc    180
   tcatcctgac gattttgctg gtatggatgc gaaccaactt cagaaacgtg gatttggatg    240
   caatggtcct tgggatgagg atgatatgca gtgccacaat cactgcaagt ctattaaggg    300
   ttacaaggga ggttattgtg ctaagggggg ctttgtttgc aagtgttact agctcgag      358
<210> 20
   <211> 362
   <212> DNA
   <213> Artificial
<220>
   <223> Plectasin encoding sequence of pMT2900 (see Example 3)
<400> 20
   ggatccacca tgcaatttac caccatcctc tccatcggta tcaccgtctt cggactgggt 60
   aagaatcact tataactagt agattaagcc aagagtattg gaactgatga taaacagcca 120
   acaccggagc ctttgcagca ccccagccgg tacccgaggc ttacgctgtt tctgatcccg 180
   aggctcatcc tgacgatttt gctggtatgg atgcgaacca acttcagaaa cgtggatttg 240
   gatgcaatgg tccttgggat gaggatgata tgcagtgcca caatcactgc aagtctatta 300
   agggttacaa gggaggttat tgtgctaagg ggggctttgt ttgcaagtgt tactagctcg 360
   ag 362
<210> 21
   <211> 34
   <212> DNA
   <213> Artificial
<220>
   <223> Primer A (see Example 5)
<400> 21
   tcttggatcc accatgcact tcaccaaggt ctcc 34
<210> 22
   <211> 33
   <212> DNA
   <213> Artificial
<220>
   <223> Primer B (see Example 5)
<400> 22
   tcttctcgag ttagaaagaa caggtgcagg tac 33
<210> 23
   <211> 386
   <212> DNA
<213> Eurotium amstelodami
<220>
   <221> CDS
   <222> (10) .. (195)
<220>
   <221> sig_peptide
   <222> (10) .. (69)
<220>
   <221> mat_peptide
   <222> (159)..(377)
<220>
   <221> Intron
   <222> (196)..(240)
<220>
   <221> CDS
   <222> (241)..(305)
<220>
   <221> Intron
   <222> (306)..(358)
<220>
   <221> CDS
   <222> (359)..(377)
<400> 23
   ggatccacc atg cac ttc acc aag gtc tcc acc att ctt ttt acc atc ttc      51
             Met His Phe Thr Lys Val Ser Thr Ile Leu Phe Thr Ile Phe
                         -45                 -40                 -35
   gcc gcc ggc atc atg gct gct ccc acc gaa gga gtc cgt gag gaa gcc        99
   Ala Ala Gly Ile Met Ala Ala Pro Thr Glu Gly Val Arg Glu Glu Ala
                   -30                 -25                 -20
   gcc cct ggc cag gag gtt tac ccc gac gaa cct cct gct tct ctg acc       147
   Ala Pro Gly Gln Glu Val Tyr Pro Asp Glu Pro Pro Ala Ser Leu Thr
               -15                 -10                 -5
   aag cgt ggc ttc gga tgt cct ggt gat gcc tac cag tgc agt gaa cac       195
   Lys Arg Gly Phe Gly Cys Pro Gly Asp Ala Tyr Gln Cys Ser Glu His
       -1  1               5                   10
   gtatgtcctg ctgctatcta gagtgaatga agctaatgaa tatag tgc agg gcc ctg     252
                                                     Cys Arg Ala Leu
                                                     15
   ggc ggt gga cgc act gga gga tac tgt gct gga cct tgg tat ttg ggt       300
   Gly Gly Gly Arg Thr Gly Gly Tyr Cys Ala Gly Pro Trp Tyr Leu Gly
       20                  25                  30
   cac cc  gtatgttgcc tagatattta attgtttata gtcctttact gatgagctta        355
   His Pro
   35
   tag t acc tgc acc tgt tct ttc taactcgag                              386
                           Thr Cys Thr Cys Ser Phe
                                       40
<210> 24
   <211> 90
   <212> PRT
   <213> Eurotium amstelodami
<400> 24
   Met His Phe Thr Lys Val Ser Thr Ile Leu Phe Thr Ile Phe Ala Ala
               -45                 -40                 -35
   Gly Ile Met Ala Ala Pro Thr Glu Gly Val Arg Glu Glu Ala Ala Pro
           -30                 -25                 -20
   Gly Gln Glu Val Tyr Pro Asp Glu Pro Pro Ala Ser Leu Thr Lys Arg
       -15                 -10                 -5              -1
   Gly Phe Gly Cys Pro Gly Asp Ala Tyr Gln Cys Ser Glu His Cys Arg
   1               5                   10                  15
   Ala Leu Gly Gly Gly Arg Thr Gly Gly Tyr Cys Ala Gly Pro Trp Tyr
               20                  25                  30
   Leu Gly His Pro Thr Cys Thr Cys Ser Phe
           35                  40

## Description

### Technical field

The present invention relates to recombinant expression of insect defensin antimicrobial peptides in *Aspergillus.*

### Background art

Defensins belong to a class of small antimicrobial peptides. They are capable of killing a broad spectrum of microorganisms, some of which are becoming increasingly resistant towards traditional antibiotics. For that reason it is also becoming more and more interesting to be capable of producing defensins in large amounts at a low cost.

Since defensins usually only comprise 30-50 amino acid residues, they are often difficult to produce efficiently by use of recombinant fermentation methods. Chemical peptide synthesis is an alternative method, but this is too expensive when peptides exceed 25-30 amino acid residues. Another complication is that defensins comprise a distinctive cysteine pattern, which is difficult to create by chemical synthesis.

Accordingly, it is an object of the present invention to provide methods for obtaining improved expression levels of insect defensin antimicrobial peptides in recombinant fermentation of *Aspergillus.*

### Summary of the invention

The inventors of the present invention have found that by inserting one or more intron sequences in a nucleic acid construct, which directs expression of an insect defensin, the recombinant expression level may be improved by more than 50% compared to using a nucleic acid construct with no intron sequences. The intron sequence(s) may be inserted anywhere in the nucleic acid construct, such as in the mature defensin encoding sequence, or even in a signal peptide encoding sequence.

Accordingly, the present invention relates to a recombinant *Aspergillus* host cell, comprising a nucleic acid construct, which nucleic acid construct comprises a foreign nucleic acid sequence encoding an insect defensin and one or more intron sequence(s).

In a second aspect, the invention relates to a method for recombinant production of an insect defensin in an *Aspergillus* host cell, which includes cultivating the *Aspergillus* host cell comprising a nucleic acid construct, which nucleic acid construct comprises a nucleic acid sequence encoding the insect defensin peptide and one or more intron sequence(s); and recovering the insect defensin peptide.

In a third aspect, the invention relates to use of a nucleic acid construct, which comprises a nucleic acid sequence encoding an insect defensin peptide and one or more intron sequence(s), for improving the recombinant expression level of the insect defensin in an *Aspergillus* host cell.

### Definitions

Antimicrobial activity: The term "antimicrobial activity" is defined herein as an activity which is capable of killing or inhibiting growth of microbial cells. In the context of the present invention the term "antimicrobial" is intended to mean that there is a bactericidal and/or a bacteriostatic and/or fungicidal and/or fungistatic effect and/or a virucidal effect, wherein the term "bactericidal" is to be understood as capable of killing bacterial cells. The term "bacteriostatic" is to be understood as capable of inhibiting bacterial growth, i.e. inhibiting growing bacterial cells. The term "fungicidal" is to be understood as capable of killing fungal cells. The term "fungistatic" is to be understood as capable of inhibiting fungal growth, i.e. inhibiting growing fungal cells. The term "virucidal" is to be understood as capable of inactivating virus. The term "microbial cells" denotes bacterial or fungal cells (including yeasts).

In the context of the present invention the term "inhibiting growth of microbial cells" is intended to mean that the cells are in the non-growing state, i.e., that they are not able to propagate.

For purposes of the present invention, antimicrobial activity may be determined according to the procedure described by Lehrer et al., Journal of Immunological Methods, Vol. 137 (2) pp. 167-174 (1991). Alternatively, antimicrobial activity may be determined according to the NCCLS guidelines from CLSI (Clinical and Laboratory Standards Institute; formerly known as National Committee for Clinical and Laboratory Standards).

Defensins having antimicrobial activity may be capable of reducing the number of living cells of *Escherichia coli* (DSM 1576) to 1/100 after 8 hours (preferably after 4 hours, more preferably after 2 hours, most preferably after 1 hour, and in particular after 30 minutes) incubation at 20°C in an aqueous solution of 25%(w/w); preferably in an aqueous solution of 10%(w/w); more preferably in an aqueous solution of 5%(w/w); even more preferably in an aqueous solution of 1%(w/w); most preferably in an aqueous solution of 0.5%(w/w); and in particular in an aqueous solution of 0.1%(w/w) of the defensins having antimicrobial activity.

Defensins having antimicrobial activity may also be capable of inhibiting the outgrowth of *Escherichia coli* (DSM 1576) for 24 hours at 25°C in a microbial growth substrate, when added in a concentration of 1000 ppm; preferably when added in a concentration of 500 ppm; more preferably when added in a concentration of 250 ppm; even more preferably when added in a concentration of 100 ppm; most preferably when added in a concentration of 50 ppm; and in particular when added in a concentration of 25 ppm.

Defensins having antimicrobial activity may be capable of reducing the number of living cells of *Bacillus subtilis* (ATCC 6633) to 1/100 after 8 hours (preferably after 4 hours, more preferably after 2 hours, most preferably after 1 hour, and in particular after 30 minutes) incubation at 20°C in an aqueous solution of 25%(w/w); preferably in an aqueous solution of 10%(w/w); more preferably in an aqueous solution of 5%(w/w); even more preferably in an aqueous solution of 1 %(w/w); most preferably in an aqueous solution of 0.5%(w/w); and in particular in an aqueous solution of 0.1%(w/w) of the defensins having antimicrobial activity.

Defensins having antimicrobial activity may also be capable of inhibiting the outgrowth of *Bacillus subtilis* (ATCC 6633) for 24 hours at 25°C in a microbial growth substrate, when added in a concentration of 1000 ppm; preferably when added in a concentration of 500 ppm; more preferably when added in a concentration of 250 ppm; even more preferably when added in a concentration of 100 ppm; most preferably when added in a concentration of 50 ppm; and in particular when added in a concentration of 25 ppm.

The Defensins have at least 20%, preferably at least 40%, more preferably at least 50%, more preferably at least 60%, more preferably at least 70%, more preferably at least 80%, even more preferably at least 90%, most preferably at least 95%, and even most preferably at least 100% of the antimicrobial activity of the defensin consisting of the amino acid sequence shown as amino acids 1 to 42 of SEQ ID NO:2.

cDNA: The term "cDNA" is defined herein as a DNA molecule which can be prepared by reverse transcription from a mature, spliced, mRNA molecule obtained from a eukaryotic cell. cDNA lacks intron sequences that are usually present in the corresponding genomic DNA. The initial, primary RNA transcript is a precursor to mRNA which is processed through a series of steps before appearing as mature spliced mRNA. These steps include the removal of intron sequences by a process called splicing. cDNA derived from mRNA lacks, therefore, any intron sequences.

Nucleic acid construct: The term "nucleic acid construct" as used herein refers to a nucleic acid molecule, either single- or double-stranded, which is isolated from a naturally occurring gene or which is modified to contain segments of nucleic acids in a manner that would not otherwise exist in nature. The term nucleic acid construct is synonymous with the term "expression cassette" when the nucleic acid construct contains the control sequences required for expression of a coding sequence.

Control sequence: The term "control sequences" is defined herein to include all components, which are necessary or advantageous for the expression of a polynucleotide encoding a defensin. Each control sequence may be native or foreign to the nucleotide sequence encoding the defensin. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleotide sequence encoding a defensin.

Operably linked: The term "operably linked" denotes herein a configuration in which a control sequence is placed at an appropriate position relative to the coding sequence of the polynucleotide sequence such that the control sequence directs the expression of the coding sequence of a defensin.

Coding sequence: When used herein the term "coding sequence" means a nucleotide sequence, which directly specifies the amino acid sequence of its protein product. The boundaries of the coding sequence are generally determined by an open reading frame, which usually begins with the ATG start codon or alternative start codons such as GTG and TTG. The coding sequence may a DNA, cDNA, or recombinant nucleotide sequence.

Expression: The term "expression" includes any step involved in the production of the defensin including, but not limited to, transcription, post-transcriptional modification, translation, post-translational modification, and secretion.

Expression vector: The term "expression vector" is defined herein as a linear or circular DNA molecule that comprises a polynucleotide encoding a defensin peptide, and which is operably linked to additional nucleotides that provide for its expression.

Host cell: The term "host cell", as used herein, includes any cell type which is susceptible to transformation, transfection, transduction, and the like with a nucleic acid construct of the present invention.

Modification: The term "modification" means herein any chemical modification of the defensin. The modification(s) can be substitution(s), deletion(s) and/or insertions(s) of the amino acid(s) as well as replacement(s) of amino acid side chain(s); or use of unnatural amino acids with similar characteristics in the amino acid sequence. In particular the modification(s) can be amidations, such as amidation of the C-terminus.

Identity: The relatedness between two amino acid sequences or between two nucleotide sequences is described by the parameter "identity".

For purposes of the present invention, the degree of identity between two amino acid sequences is determined by using the program FASTA included in version 2.0x of the FASTA program package (see W. R. Pearson and D. J. Lipman (1988), "Improved Tools for Biological Sequence Analysis", PNAS 85:2444-2448; and W. R. Pearson (1990) "Rapid and Sensitive Sequence Comparison with FASTP and FASTA", Methods in Enzymology 183:63-98). The scoring matrix used was BLOSUM50, gap penalty was -12, and gap extension penalty was -2.

The degree of identity between two nucleotide sequences is determined using the same algorithm and software package as described above. The scoring matrix used was the identity matrix, gap penalty was -16, and gap extension penalty was -4.

Alternatively, an alignment of two amino acid sequences is determined by using the Needle program from the EMBOSS package (http://emboss.org) version 2.8.0. The Needle program implements the global alignment algorithm described in Needleman, S. B. and Wunsch, C. D. (1970) J. Mol. Biol. 48, 443-453. The substitution matrix used is BLOSUM62, gap opening penalty is 10, and gap extension penalty is 0.5.

The degree of identity between an amino acid sequence of the present invention ("invention sequence"; e.g. amino acids 1 to 40 of SEQ ID NO:2) and a different amino acid sequence ("foreign sequence") is calculated as the number of exact matches in the overlap of an alignment of the two sequences, divided by the length of the "invention sequence" or the length of the "foreign sequence", whichever is the shortest. The result is expressed in percent identity.

An exact match occurs when the "invention sequence" and the "foreign sequence" have identical amino acid residues in the same positions of the overlap. The length of a sequence is the number of amino acid residues in the sequence (e.g. the length of amino acids 1 to 40 of SEQ ID NO:2 is 40).

### Detailed description

### Defensins

The defensins of the invention is any antimicrobial peptide recognized by a person skilled in the art as belonging to the insect defensin class of antimicrobial peptides. To determine if an antimicrobial peptide is an insect defensin; the amino acid sequence is preferably compared with the hidden markov model profiles (HMM profiles) of the well-known PFAM database (see Example 6).

The insect defensins may also be synthetic insect defensins sharing the characteristic features of the insect defensin class.

In an embodiment, the amino acid sequence of an insect defensin comprises 4, 5, 6, 7, 8, 9, or 10 cysteine residues, preferably 6, 7, 8, 9, or 10 cysteine residues, more preferably 6, 8, or 10 cysteine residues, and most preferably 6 or 8 cysteine residues.

Examples of insect defensins include, but are not limited to, Drosomycin, Heliomicin, γ1-purothionin, Insect defensin A, and the defensins disclosed in PCT applications WO 9953053 (RHONE POULENC AGROCHIMIE) 21.10.1999 and WO 02085934 (ENTOMED) 31.10.2002; or those set forth in the mature amino acid sequences of SEQ ID NO:2, SEQ ID NO:4, SEQ ID NO:6, SEQ ID NO:8, SEQ ID NO:10, SEQ ID NO:12, SEQ ID NO:14 and SEQ ID NO:16. An insect defensin according to the invention may furthermore comprise one or more chemical modifications compared to these amino acid sequences.

Insect-defensins may be defined as antimicrobial peptides comprising the amino acid sequence: 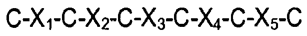 - wherein
**X₁** represents 5-16 amino acids;
**X₂** represents 3 amino acids; preferably X₂=Z₁-Z₂-Z₃, wherein
Z₁ represents any amino acid; preferably Z₁=A or H;
Z₂ represents any amino acid; preferably Z₂=A or R;
Z₃ represents any amino acid; preferably Z₃=H;
**X₃** represents 9-11 amino acids;
**X₄** represents; 4-10 amino acids;
**X₅** represents 1 amino acid; preferably X₅=V, T, I, H, K, N or L.

In an embodiment, the defensin has more than one antimicrobial activity selected from antifungal activity, antibacterial activity and antiviral activity.

A defensin may be obtained from microorganisms of any genus. For purposes of the present invention, the term "obtained from" as used herein in connection with a given source shall mean that the defensin encoded by a nucleotide sequence is produced by the source or by a strain in which the nucleotide sequence from the source has been inserted. In a preferred aspect, the defensin obtained from a given source is secreted extracellularly.

A defensin may be a fungal defensin, and more preferably a yeast defensin such as a *Candida, Kluyveromyces, Pichia, Saccharomyces, Schizosaccharomyces,* or *Yarrowia* defensin; or more preferably a filamentous fungal defensin such as an *Acremonium, Aspergillus, Aureobasidium, Cryptococcus, Filibasidium, Fusarium, Humicola, Magnaporthe, Mucor, Myceliophthora, Neocallimastix, Neurospora, Paecilomyces, Penicillium, Piromyces, Schizophyllum, Talaromyces, Thermoascus, Thielavia*, *Tolypocladium,* or *Trichoderma* defensin.

In a preferred aspect, the defensin is a *Saccharomyces carlsbergensis, Saccharomyces cerevisiae, Saccharomyces diastaticus, Saccharomyces douglasii, Saccharomyces kluyveri, Saccharomyces norbensis,* or *Saccharomyces oviformis* defensin having antimicrobial activity.

In another preferred aspect, the defensin is an *Aspergillus aculeatus, Aspergillus awamori, Aspergillus fumigatus, Aspergillus foetidus, Aspergillus japonicus, Aspergillus nidulans, Aspergillus niger, Aspergillus oryzae, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Trichoderma harzianum, Trichoderma koningii, Trichoderma longibrachiatum, Trichoderma reesei,* or *Trichoderma viride* defensin.

It will be understood that for the aforementioned species, the disclosure encompasses both the perfect and imperfect states, and other taxonomic equivalents, *e.g*., anamorphs, regardless of the species name by which they are known. Those skilled in the art will readily recognize the identity of appropriate equivalents.

Strains of these species are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), and Agricultural Research Service Patent Culture Collection, Northern Regional Research Center (NRRL).

Furthermore, such defensins may be identified and obtained from other sources including microorganisms isolated from nature (*e.g*., soil, composts, water, etc.) using the above-mentioned probes. Techniques for isolating microorganisms from natural habitats are well known in the art. The polynucleotide may then be obtained by similarly screening a genomic or cDNA library of another microorganism. Once a polynucleotide sequence encoding a defensin has been detected with the probe(s), the polynucleotide can be isolated or cloned by utilizing techniques which are well known to those of ordinary skill in the art (see, *e.g*., SAMBROOK, et al. Molecular Cloning: A Laboratory Manual. 2. edition. Cold Spring Harbor Laboratory Press, 1989, ISBN 0879693096.).

Defensins also include fused defensins or cleavable fusion defensins in which another defensin is fused at the N-terminus or the C-terminus of the defensin or fragment thereof. A fused defensin is produced by fusing a nucleotide sequence (or a portion thereof) encoding another defensin to a nucleotide sequence (or a portion thereof). Techniques for producing fusion defensins are known in the art, and include ligating the coding sequences encoding the defensins so that they are in frame and that expression of the fused defensin is under control of the same promoter(s) and terminator.

### Nucleic Acid Sequences

The present disclosure also relates to polynucleotides having a nucleotide sequence which encodes a defensin

Examples of such polynucleotides include, but are not limited to, those disclosed in PCT application WO 99/53053.

In a preferred embodiment, the nucleotide sequence is set forth in SEQ ID NO:1, SEQ ID NO:3, SEQ ID NO:5, SEQ ID NO:7, SEQ ID NO:9, SEQ ID NO:11, SEQ ID NO:13 or SEQ ID NO:15. In another preferred embodiment, the nucleotide sequence is the mature defensin coding region of SEQ ID NO:1, SEQ ID NO:3, SEQ ID NO:5, SEQ ID NO:7, SEQ ID NO:9, SEQ ID NO:11, SEQ ID NO:13 or SEQ ID NO:15. The present disclosure also encompasses nucleotide sequences which encode a defensin having the amino acid sequence of SEQ ID NO:2, SEQ ID NO:4, SEQ ID NO:6, SEQ ID NO:8, SEQ ID NO:10, SEQ ID NO:12, SEQ ID NO:14 or SEQ ID NO:16, or the mature defensin thereof, which differ from SEQ ID NO:1, SEQ ID NO:3, SEQ ID NO:5, SEQ ID NO:7, SEQ ID NO:9, SEQ ID NO:11, SEQ ID NO:13 or SEQ ID NO:15 by virtue of the degeneracy of the genetic code.

The techniques used to isolate or clone a polynucleotide encoding a defensin are known in the art and include isolation from genomic DNA, preparation from cDNA, or a combination thereof. Cloning of the polynucleotides encoding the defensins from such genomic DNA can be effected, *e.g*., by using the well known polymerase chain reaction (PCR) or antibody screening of expression libraries to detect cloned DNA fragments with shared structural features. See, *e.g*., Innis et al., 1990, PCR: A Guide to Methods and Application, Academic Press, New York. Other nucleic acid amplification procedures such as ligase chain reaction (LCR), ligated activated transcription (LAT) and nucleotide sequence-based amplification (NASBA) may be used. The polynucleotides may be cloned from a strain of *Eurotium, Aspergillus, Pseudoplectania, Crassostrea, Mesobuthus* or another or related organism and thus, for example, may be an allelic or species variant of the defensin encoding region of the nucleotide sequences.

The present disclosure also relates to polynucleotides having nucleotide sequences which have a degree of identity to the mature defensin coding sequence of SEQ ID NO:1, SEQ ID NO:3, SEQ ID NO:5, SEQ ID NO:7, SEQ ID NO:9, SEQ ID NO:11, SEQ ID NO:13 or SEQ ID NO:15 of at least 60%, preferably at least 65%, more preferably at least 70%, more preferably at least 75%, more preferably at least 80%, more preferably at least 85%, more preferably at least 90%, even more preferably at least 95%, and most preferably at least 97% identity, which encode a defensin antimicrobial polypeptide.

Modification of a nucleotide sequence encoding a defensin may be necessary for the synthesis of defensins substantially similar to the defensin. The term "substantially similar" to the defensin refers to non-naturally occurring forms of the defensin. These defensins may differ in some engineered way from the defensin isolated from its native source, *e.g*., artificial variants that differ in specific activity, thermostability, pH optimum, or the like. The variant sequence may be constructed on the basis of the nucleotide sequence presented as the defensin encoding region of SEQ ID NO:1, SEQ ID NO:3, SEQ ID NO:5, SEQ ID NO:7, SEQ ID NO:9, SEQ ID NO:11, SEQ ID NO:13 or SEQ ID NO:15 *e.g*., a subsequence thereof, and/or by introduction of nucleotide substitutions which do not give rise to another amino acid sequence of the defensin encoded by the nucleotide sequence, but which correspond to the codon usage of the host organism intended for production of the enzyme, or by introduction of nucleotide substitutions which may give rise to a different amino acid sequence. For a general description of nucleotide substitution, see, *e.g*., Ford et al., 1991, Protein Expression and Purification 2: 95-107.

It will be apparent to those skilled in the art that such substitutions can be made outside the regions critical to the function of the molecule and still result in an active defensin. Amino acid residues essential to the activity of the defensin, and therefore preferably not subject to substitution, may be identified according to procedures known in the art, such as site-directed mutagenesis or alanine-scanning mutagenesis (see, *e.g*., Cunningham and Wells, 1989, Science 244: 1081-1085). In the latter technique, mutations are introduced at every positively charged residue in the molecule, and the resultant mutant molecules are tested for antimicrobial activity to identify amino acid residues that are critical to the activity of the molecule. Sites of interaction can also be determined by analysis of the three-dimensional structure as determined by such techniques as nuclear magnetic resonance analysis, crystallography or photoaffinity labelling (see, *e.g*., de Vos et al., 1992, Science 255: 306-312; Smith et al., 1992, Journal of Molecular Biology 224: 899-904; Wlodaver et al., 1992, FEBS Letters 309: 59-64).

The present disclosure also relates to polynucleotides encoding a defensin of the invention, which hybridize under low stringency conditions, preferably medium stringency conditions, more preferably medium-high stringency conditions, even more preferably high stringency conditions, and most preferably very high stringency conditions with (i) the mature peptide encoding nucleotide sequence contained in SEQ ID NO:1, SEQ ID NO:3, SEQ ID NO:5, SEQ ID NO:7, SEQ ID NO:9, SEQ ID NO:11, SEQ ID NO:13 or SEQ ID NO:15, (ii) the cDNA sequence contained in SEQ ID NO:1, SEQ ID NO:3, SEQ ID NO:5, SEQ ID NO:7, SEQ ID NO:9, SEQ ID NO:11, SEQ ID NO:13 or SEQ ID NO:15, or (iii) a complementary strand of (i) or (ii); or allelic variants and subsequences thereof (Sambrook *et al.,* 1989, *supra*), as defined herein.

The present disclosure also relates to polynucleotides obtained by (a) hybridizing a population of DNA under low, medium, medium-high, high, or very high stringency conditions with (i) the mature defensin encoding nucleotide sequence contained in SEQ ID NO:1, SEQ ID NO:3, SEQ ID NO:5, SEQ ID NO:7, SEQ ID NO:9, SEQ ID NO:11, SEQ ID NO:13 or SEQ ID NO:15, (ii) the cDNA sequence contained in SEQ ID NO:1, SEQ ID NO:3, SEQ ID NO:5, SEQ ID NO:7, SEQ ID NO:9, SEQ ID NO:11, SEQ ID NO:13 or SEQ ID NO:15, or (iii) a complementary strand of (i) or (ii); and (b) isolating the hybridizing polynucleotide, which encodes a polypeptide having antimicrobial activity.

### Nucleic Acid Constructs

The present disclosure also relates to nucleic acid constructs comprising a polynucleotide encoding a defensin operably linked to one or more control sequences which direct the expression of the coding sequence in a suitable host cell under conditions compatible with the control sequences.

A polynucleotide encoding a defensin may be manipulated in a variety of ways to provide for expression of the defensin. Manipulation of the polynucleotide's sequence prior to its insertion into a vector may be desirable or necessary depending on the expression vector. The techniques for modifying polynucleotide sequences utilizing recombinant DNA methods are well known in the art.

The control sequence may be an appropriate promoter sequence, a nucleotide sequence which is recognized by a host cell for expression of a polynucleotide encoding a defensin. The promoter sequence contains transcriptional control sequences which mediate the expression of the defensin. The promoter may be any nucleotide sequence which shows transcriptional activity in the host cell of choice including mutant, truncated, and hybrid promoters, and may be obtained from genes encoding extracellular or intracellular polypeptides either homologous or heterologous to the host cell.

Examples of suitable promoters for directing the transcription of the nucleic acid constructs in a filamentous fungal host cell are promoters obtained from the genes for *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase (*glaA*), *Rhizomucor miehei* lipase, *Aspergillus oryzae* alkaline protease, *Aspergillus oryzae* triose phosphate isomerase, *Aspergillus nidulans* acetamidase, *Fusarium venenatum* amyloglucosidase (WO 00/56900), *Fusarium venenatum* Daria (WO 00/56900), *Fusarium venenatum* Quinn (WO 00/56900), *Fusarium oxysporum* trypsin-like protease (WO 96/00787), *Trichoderma reesei* beta-glucosidase, *Trichoderma reesei* cellobiohydrolase I, *Trichoderma reesei* endoglucanase I, *Trichoderma reesei* endoglucanase II, *Trichoderma reesei* endoglucanase III, *Trichoderma reesei* endoglucanase IV, *Trichoderma reesei* endoglucanase V, *Trichoderma reesei* xylanase I, *Trichoderma reesei* xylanase II, *Trichoderma reesei* beta-xylosidase, as well as the NA2-tpi promoter (a hybrid of the promoters from the genes for *Aspergillus niger* neutral alpha-amylase and *Aspergillus oryzae* triose phosphate isomerase); and mutant, truncated, and hybrid promoters thereof.

The control sequence may also be a suitable transcription terminator sequence, a sequence recognized by a host cell to terminate transcription. The terminator sequence is operably linked to the 3' terminus of the nucleotide sequence encoding the defensin. Any terminator which is functional in the host cell of choice may be used in the present invention.

Preferred terminators for filamentous fungal host cells are obtained from the genes for *Aspergillus oryzae* TAKA amylase, *Aspergillus niger* glucoamylase, *Aspergillus nidulans* anthranilate synthase, *Aspergillus niger* alpha-glucosidase, and *Fusarium oxysporum* trypsin-like protease.

The control sequence may also be a suitable leader sequence, a nontranslated region of an mRNA which is important for translation by the host cell. The leader sequence is operably linked to the 5' terminus of the nucleotide sequence encoding the defensin. Any leader sequence that is functional in the host cell of choice may be used in the present invention.

Preferred leaders for filamentous fungal host cells are obtained from the genes for *Aspergillus oryzae* TAKA amylase and *Aspergillus nidulans* triose phosphate isomerase.

The control sequence may also be a polyadenylation sequence, a sequence operably linked to the 3' terminus of the nucleotide sequence and which, when transcribed, is recognized by the host cell as a signal to add polyadenosine residues to transcribed mRNA. Any polyadenylation sequence which is functional in the host cell of choice may be used in the present invention.

Preferred polyadenylation sequences for filamentous fungal host cells are obtained from the genes for *Aspergillus oryzae* TAKA amylase, *Aspergillus niger glucoamylase, Aspergillus nidulans* anthranilate synthase, *Fusarium oxysporum* trypsin-like protease, and *Aspergillus niger* alpha-glucosidase.

The control sequence may also be a signal peptide coding region that codes for an amino acid sequence linked to the amino terminus of a defensin and directs the encoded defensin into the cell's secretory pathway. The 5' end of the coding sequence of the nucleotide sequence may inherently contain a signal peptide coding region naturally linked in translation reading frame with the segment of the coding region which encodes the secreted defensin. Alternatively, the 5' end of the coding sequence may contain a signal peptide coding region which is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the defensin. However, any signal peptide coding region which directs the expressed defensins into the secretory pathway of a host cell of choice may be used in the present invention.

Effective signal peptide coding regions for filamentous fungal host cells are the signal peptide coding regions obtained from the genes for *Aspergillus oryzae* TAKA amylase, *Aspergillus niger* neutral amylase, *Aspergillus niger* glucoamylase, *Rhizomucor miehei* aspartic proteinase, *Humicola insolens* cellulase, and *Humicola lanuginosa* lipase.

In a preferred aspect, the signal peptide coding region is nucleotides 1 to 69 of SEQ ID NO:1 which encode amino acids -55 to -33 of SEQ ID NO:2; nucleotides 1 to 60 of SEQ ID NO:3 which encode amino acids -48 to -29 of SEQ ID NO:4; nucleotides 1 to 60 of SEQ ID NO:5 which encode amino acids -50 to -31 of SEQ ID NO:6; nucleotides 1 to 66 of SEQ ID NO:7 which encode amino acids -22 to -1 of SEQ ID NO:8; nucleotides 1 to 72 of SEQ ID NO:9 which encode amino acids -24 to -1 of SEQ ID NO:10; nucleotides 1 to 66 of SEQ ID NO:11 which encode amino acids -22 to -1 of SEQ ID NO:12; nucleotides 1 to 66 of SEQ ID NO:13 which encode amino acids -22 to -1 of SEQ ID NO:14; or nucleotides 1 to 54 of SEQ ID NO:15 which encode amino acids -26 to -9 of SEQ ID NO:16.

The control sequence may also be a propeptide coding region that codes for an amino acid sequence positioned at the amino terminus of a polypeptide. The resultant polypeptide is known as a propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for *Bacillus subtilis* alkaline protease *(aprE), Bacillus subtilis* neutral protease *(nprT), Saccharomyces cerevisiae* alpha-factor, *Rhizomucor miehei* aspartic proteinase, and *Myceliophthora thermophila* laccase (WO 95/33836).

In a preferred aspect, the propeptide coding region is nucleotides 70 to 165 of SEQ ID NO:1 which encode amino acids -32 to -1 of SEQ ID NO:2; nucleotides 61 to 144 of SEQ ID NO:3 which encode amino acids -28 to -1 of SEQ ID NO:4; nucleotides 61 to 150 of SEQ ID NO:5 which encode amino acids -30 to -1 of SEQ ID NO:6; or nucleotides 55 to 78 of SEQ ID NO:15 which encode amino acids -8 to -1 of SEQ ID NO:16.

Where both signal peptide and propeptide regions are present at the amino terminus of a polypeptide, the propeptide region is positioned next to the amino terminus of a polypeptide and the signal peptide region is positioned next to the amino terminus of the propeptide region.

It may also be desirable to add regulatory sequences which allow the regulation of the expression of the defensin relative to the growth of the host cell. Examples of regulatory systems are those which cause the expression of the gene to be turned on or off in response to a chemical or physical stimulus, including the presence of a regulatory compound. Regulatory systems in prokaryotic systems include the *lac, tac,* and *trp* operator systems. In yeast, the ADH2 system or GAL1 system may be used. In filamentous fungi, the TAKA alpha-amylase promoter, *Aspergillus niger* glucoamylase promoter, and *Aspergillus oryzae* glucoamylase promoter may be used as regulatory sequences. Other examples of regulatory sequences are those which allow for gene amplification. In eukaryotic systems, these include the dihydrofolate reductase gene which is amplified in the presence of methotrexate, and the metallothionein genes which are amplified with heavy metals. In these cases, the nucleotide sequence encoding the defensin would be operably linked with the regulatory sequence.

### Expression Vectors

The present disclosure also relates to recombinant expression vectors comprising a polynucleotide encoding a defensin , a promoter, and transcriptional and translational stop signals. The various nucleic acids and control sequences described above may be joined together to produce a recombinant expression vector which may include one or more convenient restriction sites to allow for insertion or substitution of the nucleotide sequence encoding the defensin at such sites. Alternatively, a nucleotide sequence encoding a defensin may be expressed by inserting the nucleotide sequence or a nucleic acid construct comprising the sequence into an appropriate vector for expression. In creating the expression vector, the coding sequence is located in the vector so that the coding sequence is operably linked with the appropriate control sequences for expression.

The recombinant expression vector may be any vector (*e.g.,* a plasmid or virus) which can be conveniently subjected to recombinant DNA procedures and can bring about expression of the nucleotide sequence. The choice of the vector will typically depend on the compatibility of the vector with the host cell into which the vector is to be introduced. The vectors may be linear or closed circular plasmids.

The vector may be an autonomously replicating vector, *i.e*., a vector which exists as an extrachromosomal entity, the replication of which is independent of chromosomal replication, *e.g*., a plasmid, an extrachromosomal element, a minichromosome, or an artificial chromosome. The vector may contain any means for assuring self-replication. Alternatively, the vector may be one which, when introduced into the host cell, is integrated into the genome and replicated together with the chromosome(s) into which it has been integrated. Furthermore, a single vector or plasmid or two or more vectors or plasmids which together contain the total DNA to be introduced into the genome of the host cell, or a transposon may be used.

The vectors used for expression of the defensins preferably contain one or more selectable markers which permit easy selection of transformed cells. A selectable marker is a gene the product of which provides for biocide or viral resistance, resistance to heavy metals, prototrophy to auxotrophs, and the like.

Examples of selectable markers for use in a filamentous fungal host cell include, but are not limited to, *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), *sC* (sulfate adenyltransferase), and *trpC* (anthranilate synthase), as well as equivalents thereof. Preferred for use in an *Aspergillus* cell are the *amdS* and *pyrG* genes of *Aspergillus nidulans* or *Aspergillus oryzae* and the bar gene of *Streptomyces hygroscopicus.*

The vectors preferably contain an element(s) that permits integration of the vector into the host cell's genome or autonomous replication of the vector in the cell independent of the genome.

For integration into the host cell genome, the vector may rely on the polynucleotide's sequence encoding the defensin or any other element of the vector for integration into the genome by homologous or nonhomologous recombination. Alternatively, the vector may contain additional nucleotide sequences for directing integration by homologous recombination into the genome of the host cell at a precise location(s) in the chromosome(s). To increase the likelihood of integration at a precise location, the integrational elements should preferably contain a sufficient number of nucleic acids, such as 100 to 10,000 base pairs, preferably 400 to 10,000 base pairs, and most preferably 800 to 10,000 base pairs, which have a high degree of identity with the corresponding target sequence to enhance the probability of homologous recombination. The integrational elements may be any sequence that is homologous with the target sequence in the genome of the host cell. Furthermore, the integrational elements may be non-encoding or encoding nucleotide sequences. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication which functions in a cell. The term "origin of replication" or "plasmid replicator" is defined herein as a nucleotide sequence that enables a plasmid or vector to replicate *in vivo.*

Examples of origins of replication useful in a filamentous fungal cell are AMA1 and ANS1 (Gems et al., 1991, Gene 98:61-67; Cullen et al., 1987, Nucleic Acids Research 15: 9163-9175; WO 00/24883). Isolation of the AMA1 gene and construction of plasmids or vectors comprising the gene can be accomplished according to the methods disclosed in WO 00/24883.

More than one copy of a polynucleotide encoding a defensin may be inserted into the host cell to increase production of the gene product. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, *e.g*., Sambrook *et al.,* 1989, *supra*).

### Host Cells

The host cell (or organism) of the invention is a cell of a species of *Aspergillus.* In a most preferred embodiment, the *filamentous fungal* host cell is an *Aspergillus awamori, Aspergillus foetidus, Aspergillus japonicus, Aspergillus aculeatus, Aspergillus niger* or *Aspergillus oryzae.* In a particular embodiment the filamentous host cell is an *Aspergillus* oryzae or *Aspergillus niger.*

In a preferred embodiment of the invention the host cell is a protease deficient or **protease** minus strain.

This may e.g. be the protease deficient strain *Aspergillus oryzae* JaL 125 having the alkaline protease gene named *"alp"* deleted. This strain is described in WO 9735956 (NOVO NORDISK A/S) 02.10.1997, or EP 429490 (GENENCOR) 05.06.1991, or the TPAP free host cell, in particular a strain of *Aspergillus niger,* disclosed in WO 9614404 (NOVO NORDISK A/S) 17.05.1996. Further, also host cells, especially *A. niger* or *A. oryzae,* with reduced production of the transcriptional activator (prtT) as described in WO 0168864 (NOVOZYMES A/S) 20.09.2001 is specifically contemplated according to the invention.

### Introns

Eukaryotic genes may be interrupted by intervening sequences (introns) which must be modified in precursor transcripts in order to produce functional mRNAs. This process of intron removal is known as pre-mRNA splicing. Usually, a branchpoint sequence of an intron is necessary for intron splicing through the formation of a lariat. Signals for splicing reside directly at the boundaries of the intron splice sites. The boundaries of intron splice sites usually have the consensus intron sequences GT and AG at their 5' and 3' extremities, respectively. While no 3' splice sites other than AG have been reported, there are reports of a few exceptions to the 5' GT splice site. For example, there are precedents where CT or GC is substituted for GT at the 5' boundary. There is also a strong preference for the nucleotide bases ANGT to follow GT where N is A, C, G, or T (primarily A or T in *Saccharomyces* species), but there is no marked preference for any particular nucleotides to precede the GT splice site. The 3' splice site AG is primarily preceded by a pyrimidine nucleotide base (Py), i.e., C or T.

The number of introns that can interrupt a fungal gene ranges from one to two, three, four, five, six, seven, eight, nine, ten, eleven, twelve or more introns. They may be distributed throughout a gene or situated towards the 5' or 3' end of a gene. In *Saccharomyces cerevisiae,* introns are located primarily at the 5' end of the gene. Introns may be generally less than 1 kb in size, and usually are less than 400 bp in size in yeast and less than 100 bp in filamentous fungi.

The *Saccharomyces cerevisiae* intron branchpoint sequence 5'-TACTAAC-3' rarely appears exactly in filamentous fungal introns. Sequence stretches closely or loosely resembling TACTAAC are seen at equivalent points in filamentous fungal introns with a general consensus NRCTRAC where N is A, C, G, or T, and R is A or G. For example, the fourth position T is invariant in both the *Neurospora crassa* and *Aspergillus nidulans* putative consensus sequences. Furthermore, nucleotides G, A, and C predominate in over 80% of the positions 3, 6, and 7, respectively, although position 7 in *Aspergillus nidulans* is more flexible with only 65% C. However, positions 1, 2, 5, and 8 are much less strict in both *Neurospora crassa* and *Aspergillus nidulans.* Other filamentous fungi have similar branchpoint stretches at equivalent positions in their introns, but the sampling is too small to discern any definite trends.

### Methods and Uses

In a first aspect, the present invention provides a recombinant *Aspergillus* host cell, comprising a nucleic acid construct, which nucleic acid construct comprises a foreign nucleic acid sequence encoding an insect defensin and one or more intron sequence(s). The term "foreign nucleic acid sequence" is intended to mean a nucleic acid sequence which has been introduced from the outside (from a foreign source).

The *Aspergillus* host cell may be capable of expressing (producing) the insect defensin in an amount of at least 150%, preferably 200%, more preferably 250%, and most preferably 300% of the amount obtained when using a nucleic acid construct without an intron sequence, such as a cDNA sequence.

The *Aspergillus* host cell may be grown in YPM growth medium for 3-5 days at 30-35 degrees Celsius under suitable agitation. This and other suitable methods for cultivating filamentous fungi are well-known in the art. The *Aspergillus* host cell may be used for recombinant production of insect defensins.

In a second aspect the invention provides a method for recombinant production of an insect defensin in an *Aspergillus* host cell, which includes cultivating the *Aspergillus* host cell comprising a nucleic acid construct, which nucleic acid construct comprises a nucleic acid sequence encoding the insect defensin peptide and one or more intron sequence(s); and recovering the insect defensin peptide. Suitable recovery methods are well-known in the art.

The invention also relates to use of a nucleic acid construct, which comprises a nucleic acid sequence encoding an insect defensin peptide and one or more intron sequence(s), for improving the recombinant expression level of the insect defensin in an *Aspergillus* host cell.

The expression level of the insect defensin may be at least 50% higher, preferably at least 75% higher, more preferably at least 100% higher, even more preferably at least 125% higher, most preferably 150% higher, and in particular 200% higher compared to using a nucleic acid construct which does not comprise an intron sequence, such as a cDNA sequence. Alternatively, the expression level of the insect defensin may be at least 150%, preferably 200%, more preferably 250%, and most preferably 300% of the expression level obtained when using a nucleic acid construct without an intron sequence, such as a cDNA sequence. The expression level is measured in gram insect defensin protein per liter fermentation fluid.

In an embodiment, the nucleic acid construct contains 1, 2, 3, 4, or 5 intron sequence(s), preferably 1, 2, 3 or 4 intron sequence(s), more preferably 1, 2 or 3 intron sequence(s), even more preferably 1 or 2 intron sequence(s), and most preferably one intron sequence.

In another embodiment, the nucleic acid construct comprises a nucleic acid sequence encoding a defensin peptide and at least one, preferably at least two, more preferably at least three, and most preferably at least four intron sequences.

The intron sequence(s) may be located in the signal-, pro- or mature peptide encoding part of the nucleic acid construct . When the nucleic acid construct contains more than one intron, the introns can be located in different parts of the construct.

The intron sequence(s) may in fact be located in any part of the insect defensin gene which is transcribed into mRNA.

### Pharmaceutical Formulations

The defensins of this invention can be incorporated into a variety of pharmaceutical formulations for therapeutic administration. More particularly, the defensins can be formulated into pharmaceutical compositions by combination with appropriate, pharmaceutically acceptable carriers or diluents, and may be formulated into preparations in solid, semi-solid, liquid or gaseous forms, such as tablets, capsules, powders, granules, ointments, creams, foams, solutions, suppositories, injections, inhalants, gels, microspheres, lotions, and aerosols. As such, administration of the defensins can be achieved in various ways, including oral, buccal, rectal, parenteral, intraperitoneal, intradermal, transdermal, intracheal, etc., administration. , the defensins are systemic after administration.

The defensins can be administered alone, in combination with each other, or they can be used in combination with other known compounds (*e.g*., perforin, antiinflammatory agents, antibiotics, *etc*.) In pharmaceutical dosage forms, the defensins may be administered in the form of their pharmaceutically acceptable salts. The following methods and excipients are merely exemplary and are in no way limiting.

For oral preparations, the defensins can be used alone or in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired , with diluents, buffering agents, moistening agents, preservatives and flavoring agents.

The defensins can be formulated into preparations for injections by dissolving, suspending or emulsifying them in an aqueous or nonaqueous solvent, such as vegetable or other similar oils, synthetic aliphatic acid glycerides, esters of higher aliphatic acids or propylene glycol; and if desired, with conventional additives such as solubilizers, isotonic agents, suspending agents, emulsifying agents, stabilizers and preservatives.

The defensins can be utilized in aerosol formulation to be administered via inhalation. The defensins can be formulated into pressurized acceptable propellants such as dichlorodifluoromethane, propane, nitrogen and the like.

Furthermore, the defensins can be made into suppositories by mixing with a variety of bases such as emulsifying bases or water-soluble bases. The defensins can be administered rectally via a suppository. The suppository can include vehicles such as cocoa butter, carbowaxes and polyethylene glycols, which melt at body temperature, yet are solidified at room temperature.

Unit dosage forms for oral or rectal administration such as syrups, elixirs, and suspensions may be provided wherein each dosage unit, for example, teaspoonful, tablespoonful, tablet or suppository, contains a predetermined amount of the composition containing one or more defensins. Similarly, unit dosage forms for injection or intravenous administration may comprise the defensins in a composition as a solution in sterile water, normal saline or another pharmaceutically acceptable carrier.

The term "unit dosage form", as used herein, refers to physically discrete units suitable as unitary dosages for human and animal subjects, each unit containing a predetermined quantity of defensins calculated in an amount sufficient to produce the desired effect in association with a pharmaceutically acceptable diluent, carrier or vehicle. The specifications for the unit dosage forms depend on the particular defensin employed and the effect to be achieved, and the pharmacodynamics associated with the defensin in the host.

The pharmaceutically acceptable excipients, such as vehicles, adjuvants, carriers or diluents, are readily available to the public. Moreover, pharmaceutically acceptable auxiliary substances, such as pH adjusting and buffering agents, tonicity adjusting agents, stabilizers, wetting agents and the like, are readily available to the public.

Typical dosages for systemic administration range from 0.1 pg to 100 milligrams per kg weight of subject per administration. A typical dosage may be one tablet taken from two to six times daily, or one time-release capsule or tablet taken once a day and containing a proportionally higher content of active ingredient. The time-release effect may be obtained by capsule materials that dissolve at different pH values, by capsules that release slowly by osmotic pressure, or by any other known means of controlled release.

Those of skill will readily appreciate that dose levels can vary as a function of the specific defensin, the severity of the symptoms and the susceptibility of the subject to side effects. Some of the specific defensins are more potent than others. Preferred dosages for a given defensin are readily determinable by those of skill in the art by a variety of means. A preferred means is to measure the physiological potency of a given defensin.

The use of liposomes as a delivery vehicle is one method of interest. The liposomes fuse with the cells of the target site and deliver the contents of the lumen intracellularly. The liposomes are maintained in contact with the cells for sufficient time for fusion, using various means to maintain contact, such as isolation, binding agents, and the like. In one aspect, liposomes are designed to be aerosolized for pulmonary administration. Liposomes may be prepared with purified proteins or peptides that mediate fusion of membranes, such as Sendai virus or influenza virus, etc. The lipids may be any useful combination of known liposome forming lipids, including cationic or zwitterionic lipids, such as phosphatidylcholine. The remaining lipid will be normally be neutral or acidic lipids, such as cholesterol, phosphatidyl serine, phosphatidyl glycerol, and the like.

For preparing the liposomes, the procedure described by **KATO, .** Expression of hepatitis B virus surface antigen in adult rat liver. Co-introduction of DNA and nuclear protein by a simplified liposome method. J. biol. chem., uaryFebr vol. 266, p. 3361-3364. ISSN 0021-9258. may be used. Briefly, the lipids and lumen composition containing peptides are combined in an appropriate aqueous medium, conveniently a saline medium where the total solids will be in the range of about 1-10 weight percent. After intense agitation for short periods of time, from about 5-60 sec., the tube is placed in a warm water bath, from about 25-40°C and this cycle repeated from about 5-10 times. The composition is then sonicated for a convenient period of time, generally from about 1-10 sec. and may be further agitated by vortexing. The volume is then expanded by adding aqueous medium, generally increasing the volume by about from 1-2 fold, followed by shaking and cooling. This method allows for the incorporation into the lumen of high molecular weight molecules.

### Formulations with Other Active Agents

For use in the subject methods, the defensins may be formulated with other pharmaceutically active agents (such as steroids), which are well-known in the art, particularly other antimicrobial agents. Other agents of interest include a wide variety of antibiotics, as known in the art. Classes of antibiotics include penicillins, e.g. penicillin G, penicillin V, methicillin, oxacillin, carbenicillin, nafcillin, ampicillin, etc.; penicillins in combination with beta-lactamase inhibitors, cephalosporins, e.g. cefaclor, cefazolin, cefuroxime, moxalactam, etc.; carbapenems; monobactams; aminoglycosides; tetracyclines; macrolides; lincomycins; polymyxins; sulfonamides; quinolones; cloramphenical; metronidazole; spectinomycin; trimethoprim; vancomycin; etc.

Anti-mycotic agents are also useful, including polyenes, e.g. amphotericin B, nystatin; 5-flucosyn; and azoles, e.g. miconazol, ketoconazol, itraconazol and fluconazol. Antituberculotic drugs include isoniazid, ethambutol, streptomycin and rifampin. Cytokines may also be included in a formulation of the defensins of the invention, e.g. interferon gamma, tumor necrosis factor alpha, interleukin 12, etc.

The present invention is further described by the following examples which should not be construed as limiting the scope of the invention.

### Examples

Chemicals used as buffers and substrates were commercial products of at least reagent grade.

### Example 1

### Expression of intron containing defensin encoding sequence in Aspergillus oryzae

The 361 bp BamH1-Xho1 digested PCR product amplified from a *P*. *nigrella* cDNA library (SEQ ID NO:6 from
WO 03044049 (NOVOZYMES A/S). 2003-05-30.
) was cloned into an *Aspergillus* expression vector, as previously described in WO 03/044049, to give plasmid pMT2549. The sequence of the intron in the Plectasin encoding sequence of pMT2549 was modified by using standard in vitro mutagenesis and SOE to introduce a single extra base thereby creating a restriction site within the intron. The resulting intron-containing (59 bp) Plectasin encoding sequence is shown as SEQ ID NO:17. The corresponding expression plasmid was named pMT2647.

pMT2647 was transformed into *Aspergillus oryzae* BECh2 as previously described in WO 03/044049. In the first place, 14 individual transformants were twice reisolated, grown on YPM medium (1% yeast extract, 2% bacto peptone and 2% maltose) and finally 10 µL samples were analysed on SDS gels as previously described in WO 03/044049. For some of the transformants the amount of plectasin produced appeared from the staining intensities to considerably surpass the estimated 50 mg/L maximal level previously obtained under these growth conditions for transformants of *A*. *oryzae* with the expression plasmid encoding the intron-free cDNA encoding Plectasin (see
WO 03/044049).

While several of the 14 pMT2549 transformants appeared to produce higher levels of Plectasin than did the best of the 30 intron-less transformants previously analysed in WO 03/044049, it should be kept in mind that each acetamide selected transformant represents an individual transformation and integration event resulting in considerable differences in yields between individual transformants. Such yield differences are well known to occur in systems based on non-homologous recombination, and are generally considered to be a consequence of the random integration locus and the number of expression plasmid integrated. It was therefore decided to compare expression also from a single copy of an expression vector integrated at a defined locus (see Example 2).

### Example 2

### Expression of defensin in Asperkillus oryzae from defined single copy integrants

To compare directly the expression in *A. oryzae* of Plectasin from intron-less cDNA to expression from the intron-containing Plectasin encoding sequence, these were transferred from pMT2548 (see WO 03/044049) and pMT2549 (above), respectively, on approx. 1.1 kb BamH1-Xba1 fragments to the BamH1-Xba1 fragment of a vector based on pJaL485 (8.3 kb). (see Example 3 in
WO 03008575 (NOVOZYMES A/S). 2003-01-30.
). pJaL485 contains for selection only the part of the *A*. *oryzae* niaD gene encoding the C-terminal part of nitrate reductase. Using a host strain such as JaL507, a derivative of JaL294 (see Example 8 in WO 03/008575) containing a deletion in this part of the niaD gene, a functional nitrate reductase, and thus the ability to grow on nitate as nitrogen source, can be restored only by homologous recombination. It has been found that most transformants selected in this way are indeed single copy integrants resulting from a single homologous cross-over. Tandem integrants at the niaD site do occur at a lower frequency. The intron-less and intron-containing pJaL485 derived expression plasmids were named pMT2777 and pMT2836, respectively. For each of these plasmids 4 transformants of *A*. *oryzae* JaL507 were selected and shown by Southern analysis to contain a single copy of the transforming plasmid integrated homologously at the niaD locus. The pMT2777 derived *A*. *oryzae* transformants were named MT2882-2885 and the pMT2836 derived transformants were MT2886-2889. Each of the transformants MT2882-2889 were grown on YPM as described above and 10 µL samples from culture supernatants after 3 and after 7 days of growth were analysed by PAGE SDS gels as above. The result clearly shows very little spread among the transformants with each plasmid (as expected since they should be independent but identical strains). On the other hand it is evident that the expression level in MT2886-2889 (intron-containing) is higher than in MT2882-2885 (intron-less).

Relative Plectasin levels in the day 7 samples of MT2882-2889 have also been determined by an ELISA assay (see Example 4).

### Example 3

### Increased expression of defensin from genes containing intron at different position and/or different intron

To facilitate transfer of sequences encoding Plectasin variants from e.g. *E*. *coli* and *S. cerevisiae* vectors to *Aspergillus* expression vectors, it was decided to relocate the Plectasin intron from originally being positioned in the sequence encoding mature Plectasin to a position in the prepro-peptide encoding sequence. To do this, mutations were introduced in the intron-less Plectasin encoding sequence by in vitro mutagenesis resulting in a MluN1 site being located in the signal peptide encoding sequence. It was only possible to introduce the MluN1 site by allowing an amino acid change in the signal peptide (Leu17 changed to Ala). This amino acid change is not predicted to impair signal peptide function according to commonly used signal prediction programs. The sequence of the intron-less MluN 1 contain-ing, Plectasin encoding sequence is shown as SEQ ID NO:18; the corresponding *Aspergillus* expression plasmid was called pMT2898.

An intron derived from the second intron of A. *niger* glucoamylase and constructed such that it could be excised from a plasmid pMT2374 as a 55 bp SnaB1-Pvu2 (see Example 1 in
WO 03104457 (NOVOZYMES A/S). 2003-12-18.
) was inserted in the MluN site of pMT2898 to give pMT2899 in which the intron was verified to be inserted in the proper orientation. The sequence of the intron containing, Plectasin encoding sequence of pMT2899 is shown as SEQ ID NO:19.

Also, the intron originally present in the sequence encoding mature Plectasin was made synthetically such that it could likewise be moved as a SnaB1-Pvu2 fragment. This was possible only by altering the last but two bases of the intron from a T to a C. This intron was also transferred to the MluN1 site of pMT2898 to give pMT2900 in which the correct intron orientation was verified by sequencing. The sequence of the intron-containing, Plectasin encoding sequence of pMT2900 is shown as SEQ ID NO:20.

Plasmids pMT2898, 2899 and 2900 were transformed into *A. oryzae* BECh 2 selecting for growth on acetamide. Approximately 20 transformants with each plasmid were twice reisolated and grown on YPM as described above. From SDS PAGE gels of the supernatants it was evident that the average Plectasin expression level was considerably higher for transformants with the intron containing constructs pMT2899 and pMT2900 compared to the intron-less construct pMT2898. Again, since expression levels between individual acetamide selected transformants vary widely, it was decided to transfer the expression cassettes of MT2898-2900 to the niaD based expression vector derived from pJaL485 as described above in Example 2. The resulting expression plasmids were pMT2901, pMT2902, and pMT2903 corresponding to pMT2898, pMT2899 and pMT2900, respectively. pMT2901-2903 were transformed into the niaD defective host JaL507, as described above in Example 2. A number of transformants were reisolated for each of the transformations. The transformants were grown on YPM and supernatants run on SDS page gels as above. Also in this case it is obvious that the expression level is higher for the intron-containing constructs pMT2902 and pMT2903 than it is for transformants with the construct pMT2901. It remains to be proven by Southern analysis which transformants are indeed single copy integrants, but from experience the majority of nitrate selected transformants in this set up can be shown to be single copy integrants, even if tandem integration does occur. Two transformants were selected as representative strains for each of pMT2901 (strains MT2946 and MT2947), pMT2902 (strains MT2948 and MT2949), and pMT2903 (strains MT2952 and MT2953).

ELISA quantification of Plectasin was also done for MT2946-2949 (see Example 4).

### Example 4

### Competitive ELISA - Estimation of Plectasin yields in Aspergillus oryzae fermentations

Using rabbit polyclonal antibody generated against purified Plectasin, an indirect competitive ELISA was applied to estimate yields in *Aspergillus oryzae* fermentation broths. This type of assay is a standard procedure generally applied to quantify amounts of a given protein/peptide, given that an antiserum has been generated.

The following material and buffers were used:
- Plectasin - a defensin described in PCT application WO 03/044049;
- F96 MaxiSorp Plate (Nunc, Cat. no.: 439454);
- F96 Microwell Plate (Nunc, Cat. no.: 269787);
- Skim milk powder (MERCK, Cat. no.: 1.15363.0500);
- Tween 20 (MERCK, Cat. no.: 8.22184.0500);
- Plectasin specific rabbit polyclonal antibody (Novozymes);
- Swine Anti-Rabbit Immunoglobulins/HRP (DAKO, P0448);
- TMB Plus, Ready-to-Go (KemEnTek, Cat. no.: 4390);
- Sulfuric acid (MERCK, Cat. no.: 1.00731.1000).
- PBS, pH 7.2,1L:
   - 8.00 g NaCl,
   - 0.20 g KCI,
   - 1.04 g K₂HPO₄,
   - 0.32 g KH₂PO₄;
- Blocking buffer: PBS, 2 % Skim milk;
- Washing buffer: PBS, 0.05% Tween 20;
- Dilution buffer: PBS, 0.5 % Skim milk, 0.05 % Tween 20.

Briefly, undiluted and serial 2-fold dilutions of culture broths were pre-incubated with 1:1000 polyclonal antibody in dilution buffer. Upon incubation for 2 hours at room temperature, these samples were transferred to Plectasin pre-coated plates (coated with 0.1 µg/ml Plectasin and residual binding blocked using blocking buffer). Upon 1 hour incubation at room temperature, bound antibody was detected using a secondary antibody (Swine anti rabbit - HRP) and thereafter a chromogen (TMB Plus). Detection of Plectasin bound antibody was measured through absorbance at 450 nm, which resulted in a titration curve of antibody binding.

Throughout the assay, plates were washed using washing buffer, and substance dilutions made as described by the manufacturer (DAKO & KemEnTek).

Interpretations: No detection of antibody bound to the well means that complete binding (inhibition) of antibody to unknown fermentation broth has occurred; whereas maximum absorbance measured equals absence of inhibition by the competitor (unknown fermentation broth). In this way we were able to estimate the amounts (diluted broths) that were needed to inhibit 50% of the binding to the wells. The results are depicted in the table below. Results were calculated relatively to yields measured by fermentation broths with no intron (MT2882, MT2883, MT2884 and MT2885). The results are shown in Table 1.

The results show that by using gene constructs containing an intron, the expression levels increased to about 330% of the expression levels obtained by using gene constructs with no intron.

**Table 1**

| **Intron** | **Culture ID** | **Relative yield** | **Average yield** |
|---|---|---|---|
| - | MT2882, day 7 | 106 | 100 |
| - | MT2883, day 7 | 108 | |
| - | MT2884, day 7 | 96 | |
| - | MT2885, day 7 | 91 | |
| + | MT2886, day 7 | 335 | **330** |
| + | MT2887, day 7 | 368 | |
| + | MT2888, day 7 | 310 | |
| + | MT2889, day 7 | 320 | |
| - | MT2946 | 77 | 89 |
| - | MT2947 | 100 | |
| + | MT2948 | 268 | **331** |
| + | MT2949 | 394 | |

### Example 5

### Increased expression of defensin from Eurotium Amstelodami

A defensin encoding cDNA from *Eurotium amstelodami* was earlier identified and the defensin was named Eurocin (see Examples of
DK 2005/000725 (NOVOZYMES A/S).
; or SEQ ID NO:3). The cDNA was expressed in *A*. *oryzae* to give the active Eurocin defensin peptide. In order to increase the expression level, an expression construct containing the genomic DNA sequence (including two introns) was made.

Genomic DNA from *E*. *amstelodami* was prepared using a standard method for preparation of fungal genomic DNA. Approximately 50 ng of genomic DNA was used as template in a PCR reaction with the following primers.

Primer A:
TCTTGGATCCACCATGCACTTCACCAAGGTCTCC (SEQ ID NO:21)

Primer B:
TCTTCTCGAGTTAGAAAGAACAGGTGCAGGTAC (SEQ ID NO:22)

10 pmoles of each primer was used in a 50 µl reaction volume. The annealing temperature was 55 degrees Celsius and extension at 72 degrees Celsius for 1 minute. A total of 35 cycles were run using the Expand High Fidelity PCR System (Roche). The PCR product was digested with BamH1 and Xho1 which cut in the overhangs introduced by the PCR primers. The digest was run on a 2% agarose gel and a band of approximately 400 bp was isolated. The isolated band was ligated into BamH1-Xho1 digested plasmid pMT2786 (see Example 2 of PCT/DK2005/000725) and transformed into *E*. *coli* MT173 selecting for leucine prototrophy. The BamH1-Xho1 insert was sequenced and shown to encode the prepro-Eurocin sequence corresponding to the cDNA previously characterized (see PCT/DK2005/000725 or SEQ ID NO:3), but also containing two intron sequences of 45 and 53 bases, respectively. The sequence and structure of the BamH1-Xho1 insert is shown in SEQ ID NO:23. The *Aspergillus* expression plasmid containing the insert was named pMT2945.

pMT2945 was transformed into *A*. *oryzae* BECh2 as described in Example 1, and 20 transformants were reisolated and fermented as in Example 1. Supernatants were analyzed on SDS gels also as described above. As previously, parallel experiments were also made using transformants with the Eurocin cDNA based expression plasmid pMT2935 (see Example 2 of PCT/DK2005/000725).

The transformants based on the cDNA construct pMT2935 and the intron containing construct pMT2945 all yielded distinct bands corresponding to Eurocin in size on the SDS gels.

Transformants based on the intron containing genomic construct pMT2945 were estimated to yield 300-400% of the expression level obtained with the transformants based on the cDNA construct pMT2935.

### Example 6

### Using the HMM files from the PFAM database to id entify a defensin

Sequence analysis using hidden markov model profiles (HMM profiles) may be carried out either online on the Internet or locally on a computer using the well-known HMMER freely available software package. The current version is HMMER 2.3.2 from October 2003.

The HMM profiles may be obtained from the well-known PFAM database. The current version is PFAM 16.0 from November 2004. Both HMMER and PFAM are available for all computer platforms from e.g. Washington University in St. Louis (USA), School of Medicine (http://pfam.wustl.edu and http://hmmer.wustl.edu).

If a query amino acid sequence, or a fragment thereof, belongs to one of the following five PFAM families, the amino acid sequence is a defensin according to the present invention:
- Defensin_beta or "Beta Defensin", accession number: PF00711;
- Defensin_propep or "Defensin propeptide", accession number: PF00879;
- Defensin_1 or "Mammalian defensin", accession number: PF00323;
- Defensin_2 or "Arthropod defensin", accession number: PF01097;
- Gamma-thionin or "Gamma-thionins family", accession number: PF00304.

An amino acid sequence belongs to a PFAM family, according to the present invention, if it generates an E-value which is greater than 0.1, and a score which is larger or equal to zero, when the PFAM database is used online, or when the hmmpfam program (from the HMMER software package) is used locally.

When the sequence analysis is carried out locally using the "hmmpfam" program, it is necessary to obtain (download) the HMM profiles from the PFAM database. Two profiles exist for each family; "xxx-ls.hmm" for glocal searches, and "xxx_fs.hmm" for local searches ("xxx" is the name of the family). That makes a total of ten profiles for the five families mentioned above.

These ten profiles may be used individually, or joined (appended) into a single profile (using a text editor - the profiles are ASCII files) that could be named e.g. "defensin.hmm". A query amino acid sequence can then be evaluated by using the following command line:
hmmpfam -E 0.1 defensin.hmm sequence_file
   - wherein "sequence_file" is a file with the query amino acid sequence in any of the formats recognized by the HMMER software package.

If the score is larger or equal to zero (0.0), and the E-value is greater than 0.1, the query amino acid sequence is a defensin according to the present invention.

The PFAM database is further described in
**BATEMAN**,. The Pfam Protein Families Database. Nucleic acids res., ary Janu vol. 32, p. D138-D141. ISSN 0305-1048.

## Claims

1. A recombinant *Aspergillus* host cell, comprising a nucleic acid construct, which nucleic acid construct comprises a foreign nucleic acid sequence encoding an insect defensin and one or more intron sequence(s).

2. The *Aspergillus* host cell of claim 1, which is an *Aspergillus niger* or *Aspergillus oryzae* host cell.

3. A method for recombinant production of an insect defensin in an *Aspergillus* host cell, which includes cultivating the *Aspergillus* host cell comprising a nucleic acid construct, which nucleic acid construct comprises a nucleic acid sequence encoding the insect defensin peptide and one or more intron sequence(s); and recovering the insect defensin peptide.

4. The method of claim 3, wherein the *Aspergillus* host cell is an *Aspergillus niger* or *Aspergillus oryzae* host cell.

5. The method of any of claims 3-4, which includes selecting a host cell exhibiting a production of the defensin in an amount of at least 150% of the amount obtained when using a nucleic acid construct without an intron sequence.

6. Use of a nucleic acid construct, which comprises a nucleic acid sequence encoding an insect defensin peptide and one or more intron sequence(s), for improving the recombinant expression level of the insect defensin in an *Aspergillus* host cell.

7. The use according to claim 6, wherein the *Aspergillus* host cell is an *Aspergillus niger* or *Aspergillus oryzae* host cell.

## Patentansprüche

1. Rekombinante Aspergillus-Wirtszelle, umfassend ein Nukleinsäurekonstrukt, wobei das Nukleinsäurekonstrukt eine fremde Nukleinsäuresequenz umfasst, die ein Insekten-Defensin und eine oder mehrere Intronsequenz(en) kodiert.

2. Aspergillus-Wirtszelle nach Anspruch 1, die eine Wirtszelle von Aspergillus niger oder Aspergillus oryzae ist.

3. Verfahren zur rekombinanten Herstellung eines Insekten-Defensins in einer Aspergillus-Wirtszelle, welches das Kultivieren der Aspergillus-Wirtszelle, die ein Nukleinsäurekonstrukt umfasst, einschließt, wobei das Nukleinsäurekonstrukt eine Nukleinsäuresequenz umfasst, die ein Insekten-Defensinpeptid und eine oder mehrere Intronsequenz(en) kodiert; und Gewinnen des Insekten-Defensinpeptids.

4. Verfahren nach Anspruch 3, wobei die Aspergillus-Wirtszelle eine Wirtszelle von Aspergillus niger oder Aspergillus oryzae ist.

5. Verfahren nach einem beliebigen der Ansprüche 3-4, welches das Auswählen einer Wirtszelle, die eine Herstellung des Defensins in einer Menge von mindestens 150% der Menge aufweist, die erhalten wird, wenn ein Nukleinsäurekonstrukt ohne eine Intronsequenz verwendet wird, einschließt.

6. Verwendung eines Nukleinsäurekonstrukts, das eine Nukleinsäuresequenz umfasst, die ein Insekten-Defensinpeptid und eine oder mehrere Intronsequenz(en) kodiert, zum Verbessern des rekombinanten Expressionsspiegels des Insekten-Defensins in einer Aspergillus-Wirtszelle.

7. Verwendung nach Anspruch 6, wobei die Aspergillus-Wirtszelle eine Wirtszelle von Aspergillus niger oder Aspergillus oryzae ist.

## Revendications

1. Cellule hôte recombinante d*'Aspergillus,* comprenant un construit d'acide nucléique, lequel construit d'acide nucléique comprend une séquence d'acide nucléique étrangère codant pour une défensine d'insecte et une ou plusieurs séquence(s) intron(s).

2. Cellule hôte d*'Aspergillus* selon la revendication 1, laquelle est une cellule hôte d*'Aspergillus niger* ou *d'Aspergillus oryzae.*

3. Procédé de production recombinante d'une défensine d'insecte dans une cellule hôte d*'Aspergillus,* lequel inclut la culture de la cellule hôte d*'Aspergillus* comprenant un construit d'acide nucléique, lequel construit d'acide nucléique comprend une séquence d'acide nucléique codant pour le peptide défensine d'insecte et une ou plusieurs séquence(s) intron(s) ; et la récupération du peptide défensine d'insecte.

4. Procédé selon la revendication 3, dans lequel la cellule hôte d*'Aspergillus* est une cellule hôte d*'Aspergillus niger* ou *d'Aspergillus oryzae.*

5. Procédé selon l'une quelconque des revendications 3-4, lequel inclut la sélection d'une cellule hôte présentant une production de la défensine en quantité d'au moins 150% de la quantité obtenue en utilisant un construit d'acide nucléique sans séquence intron.

6. Utilisation d'un construit d'acide nucléique, lequel comprend une séquence d'acide nucléique codant pour un peptide défensine d'insecte et une ou plusieurs séquence(s) intron(s), pour améliorer le niveau d'expression recombinante de la défensine d'insecte dans une cellule hôte d*'Aspergillus.*

7. Utilisation selon la revendication 6, dans laquelle la cellule hôte d*'Aspergillus* est une cellule hôte d*'Aspergillus niger* ou *d'Aspergillus oryzae.*
